(19) **Europäisches Patentamt**
**European Patent Office**
**Office européen des brevets**

(11) **EP 2 912 674 B1**

(12) **FASCICULE DE BREVET EUROPEEN**

(45) Date de publication et mention de la délivrance du brevet:
**24.08.2016 Bulletin 2016/34**

(21) Numéro de dépôt: **13779253.7**

(22) Date de dépôt: **17.09.2013**

(51) Int Cl.:
*H01G 11/36* $^{(2013.01)}$     *G06F 19/00* $^{(2011.01)}$

(86) Numéro de dépôt international:
**PCT/FR2013/052137**

(87) Numéro de publication internationale:
**WO 2014/068204 (08.05.2014 Gazette 2014/19)**

(54) **PROCÉDÉ POUR DÉTERMINER L'ÉNERGIE TOTALE POUR DÉSOLVATER UN COUPLE ANION-CATION ET L'INSÉRER DANS L'ÉLECTRODE D'UN SUPERCONDENSATEUR**

VERFAHREN ZUR BESTIMMUNG DER GESAMTENERGIE ZUM DESOLVATISIEREN EINES ANIONEN-KATIONEN-PAARS UND EINSATZ IN DIE ELEKTRODE EINES SUPERKONDENSATORS

METHOD FOR DETERMINING THE TOTAL ENERGY FOR DESOLVATING AN ANION-CATION PAIR AND INSERTING IT INTO THE ELECTRODE OF A SUPERCAPACITOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.10.2012 FR 1202895**

(43) Date de publication de la demande:
**02.09.2015 Bulletin 2015/36**

(73) Titulaire: **IFP Énergies nouvelles**
**92852 Rueil-Malmaison Cedex (FR)**

(72) Inventeurs:
- **JOVER AZPURUA, Julio**
  **F-92400 Courbevoie (FR)**
- **DE BRUIN, Theodorus**
  **F-75012 Paris (FR)**
- **TOULHOAT, Herve**
  **F-95220 Herblay (FR)**

(56) Documents cités:
**EP-A1- 2 306 476**

- **SHIM Y ET AL: "Nanoporous carbon supercapacitors in an ionic liquid: A computer simulation study", ACS NANO, vol. 4, no. 4, 27 avril 2010 (2010-04-27), pages 2345-2355, XP055074009, ISSN: 1936-0851, DOI: 10.1021/nn901916m**
- **HEYES D M: "Electrostatic potentials and fields in infinite point charge lattices", JOURNAL OF CHEMICAL PHYSICS, vol. 74, no. 3, 1 février 1981 (1981-02-01), pages 1924-1929, XP009171718, ISSN: 0021-9606, DOI: 10.1063/1.441285**
- **CÉLINE M ET AL: "On the molecular origin of supercapacitance in nanoporous carbon electrodes", NATURE MATERIALS, vol. 11, no. 4, 4 mars 2012 (2012-03-04), pages 306-310, XP055074167, ISSN: 1476-1122, DOI: 10.1038/nmat3260**

**Description**

La présente invention concerne le domaine des supercondensateurs notamment, et de façon générale elle concerne un procédé pour simuler les interactions électrostatiques entre un adsorbant électriquement chargé et un adsorbat électriquement chargé.

En particulier, l'invention est un procédé pour déterminer l'énergie totale pour désolvater un couple anion-cation d'un liquide ionique et pour insérer ce couple dans l'électrode d'un supercondensateur. L'invention, permet également de réaliser un criblage de couples matériaux/cation-anion du liquide ionique de supercondensateurs. En effet, cette énergie totale, étant liée à la capacité d'un supercondensateur, est un bon critère de criblage.

Le stockage d'énergie électrique est un axe important notamment dans le cadre du développement des véhicules hybrides et/ou électriques. Les supercondensateurs (ou condensateurs électrochimiques) se distinguent des condensateurs conventionnels par une puissance (très) élevée. Ils sont particulièrement adaptés pour des applications qui requièrent des pulses d'énergie sur des temps très courts, de l'ordre de la minute.

Un supercondensateur est constitué de deux électrodes poreuses, généralement en charbon actif et imprégnées d'électrolyte, qui sont séparées par une membrane isolante et poreuse (pour assurer la conduction ionique). La couche double électrique se développe sur chaque interface électrode-électrolyte, de sorte que l'on peut voir schématiquement un supercondensateur comme l'association en série de deux condensateurs, l'un à l'électrode positive et l'autre à l'électrode négative. En raison des lois d'association des condensateurs, la capacité de l'ensemble en série est toujours inférieure à la plus faible de ces deux capacités.

Contrairement aux batteries, dans les supercondensateurs il n'y a pas de réaction chimique responsable du transport des électrons, mais ils fonctionnent sur un phénomène physique.

Pendant la période de charge, on applique un potentiel sur les deux électrodes : les ions dans l'électrolyte sont séparés et forment une double couche électrochimique : l'électrode négative attire les ions positifs et l'électrode positive les ions négatifs. Dès que l'on met un collecteur de courant, le condensateur se décharge et la double couche est détruite.

La capacité (C) du condensateur est déterminée par la quantité de charges stockée (Q) et la différence de potentiel appliquée ($\Delta V$) selon :

$$C = \frac{Q}{\Delta V} \quad . \tag{1.1}$$

La différence de potentiel s'exprime en fonction de la charge :

$$\Delta V = Ed = \frac{Q}{\varepsilon \, A} d \quad ; \tag{1.2}$$

où E est la valeur du champ électrique, $\varepsilon$ la permittivité du milieu, A est l'aire de l'interface électrode/électrolyte et d est l'épaisseur de la double couche.

Il vient donc :

$$C = \frac{\varepsilon \, A}{d} \quad . \tag{1.3}$$

Pour une électrode, sa capacité croît quand on augmente l'aire de l'interface et lorsque l'on diminue l'épaisseur de la double couche. L'épaisseur peut être diminuée quand les ions sont dérobés de leur molécules de solvant (eau, acétonitrile) ou leur contre ion. La famille des liquides ioniques (sels liquides à une température inférieure à 100 °C) montre cette capacité/propriété de pouvoir se dérober de leur contre ion.

Afin d'optimiser la capacité des supercapaciteurs utilisant un liquide ionique de nombreux couples matériaux/cation-anion du liquide ionique doivent être testés.

Une méthode pour réaliser ce criblage consiste à déterminer pour chaque couple l'énergie totale ($\Delta E_{tot}$) pour désolvater un couple de liquide ionique (anion+cation) et l'adsorber dans les pores de l'électrode. Il s'avère que cette énergie totale est inversement proportionnelle à la capacité.

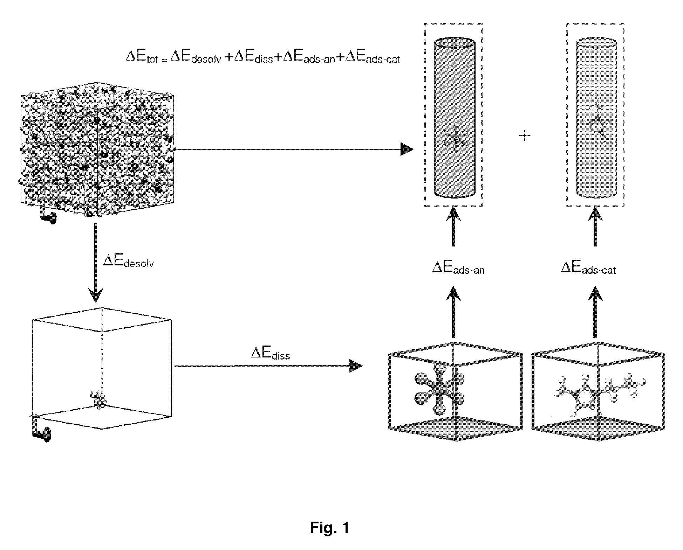

Afin de faciliter le calcul de $\Delta E_{tot}$, il est connu d'utiliser un cycle thermodynamique qui permet de couper $\Delta E_{tot}$ en trois termes d'énergie dont la somme est égale à $\Delta E_{tot}$ (figure 1) :

1. l'énergie de désolvatation ($\Delta E_{desolv}$)

Elle correspond à l'énergie à apporter pour faire sortir un couple de liquide ionique (cation+anion) de la phase condensée (liquide ionique pur ou dissout dans un solvant tel que l'acétonitrile) à la phase gaz. Cette technique est notamment décrite dans le document suivant : T. Köddermann, D. Paschek, R. Ludwig, ChemPhysChem 2008, 9, 549.

2. l'énergie de dissociation ($\Delta E_{diss}$)

Elle représente l'énergie nécessaire pour dissocier le cation et l'anion. Cette technique est notamment décrite dans le document suivant : Shimizu, K.; Tariq, M.; Costa Gomes, M. F.; Rebelo, L. P. N.; Canongia Lopes, J. N. The journal of Physical Chemistry. B 2010, 114, 5831-4.

3. l'énergie d'adsorption ($\Delta E_{ads}$)

Elle est libérée quand on fait rentrer l'ion dans les pores de l'électrode. Dans le cadre d'un supercondensateur, on calcule l'énergie d'adsorption de l'anion ($\Delta E_{ads-an}$) et l'énergie d'adsorption du cation ($\Delta E_{ads-cat}$).

La figure 1 illustre cette stratégie qui permet de calculer l'énergie totale nécessaire pour enlever un cation et un anion de leur phase condensée et de les adsorber dans les pores de l'électrode.

Cependant, la détermination de l'énergie d'adsorption en suivant ce cycle thermodynamique, requiert la prise en compte des interactions électrostatiques de courte et longue portée entre les ions (appelés « guest ») et l'électrode (hôte). Or, les contraintes imposées par le formalisme du calcul empêchent la prise en compte de ces interactions, qui sont pourtant essentielles.

Ainsi, l'objet de l'invention concerne un procédé pour déterminer l'énergie d'adsorption de couples anion-cation dans un supercondensateur, tout en prenant en compte les interactions électrostatiques entre les ions et l'électrode.

L'invention concerne également un procédé de criblage de couples anion-cation, et un procédé de dimensionnement des pores des électrodes d'un supercondensateur.

Une étude de la capacité spécifique d'un supercondensateur employant des simulations dynamiques moléculaires a déjà été publiée dans SHIM Y et al, ACS Nano, vol. 4, no. 4, le 27 avril 2010, pages 2345-2355, ISSN 1936-0851.

**Le procédé selon l'invention**

De façon générale, l'invention concerne un procédé pour déterminer l'énergie d'adsorption entre un adsorbant électriquement chargé et un adsorbat électriquement chargé, prenant en compte des interactions électrostatiques entre l'adsorbat et l'adsorbant. Le procédé comporte les étapes suivantes :

- on construit une boite de simulation (BSA) comportant ledit adsorbant (A, B) et ledit adsorbat (i+, i-), ainsi qu'un autre adsorbant (A', B') du même type mais de charge opposée et un autre adsorbat (i'+, i'-) du même type mais de charge opposée, de façon à ce que ladite boite de simulation ait une charge nulle ;
- on détermine l'énergie d'adsorption desdits adsorbats (i+, i- et i') dans ladite première boite de simulation (BSA) au moyen d'une simulation moléculaire et de la méthode Ewald, et on en déduit l'énergie d'adsorption dudit adsorbat (i+, i-) sur ledit adsorbant (A, B).

Selon l'invention, l'adsorbant peut être une zéolithe, ou un nanotube ou une enzyme ou une électrode (A, B), et l'adsorbat peut être un ion (i+,i-) ou une protéine.

Selon un mode de réalisation, on détermine l'énergie d'adsorption d'un couple anion-cation d'un liquide ionique, sur deux électrodes d'un supercondensateur, en prenant en compte des interactions électrostatiques entre les ions et l'électrode en réalisant les étapes suivantes :

- on construit une première boite de simulation (BSA) comportant ladite électrode positive A et au moins un anion, ainsi qu'une électrode A' de charge opposée et au moins un ion de charge opposée, de façon à ce que ladite boite de simulation ait une charge nulle ;

- on construit une seconde boite de simulation (BSB) comportant ladite électrode négative B et au moins un cation, ainsi qu'une électrode B' de charge opposée et au moins un ion de charge opposée, de façon à ce que ladite boite de simulation ait une charge nulle ;

- on détermine une contribution électrostatique de l'énergie d'adsorption desdits ions dans ladite première boite de simulation (BSA) au moyen de la méthode Ewald, et on en déduit l'énergie d'adsorption des anions ;

- on détermine une contribution électrostatique de l'énergie d'adsorption desdits ions dans ladite seconde boite de simulation (BSB) au moyen de la méthode Ewald, et on en déduit l'énergie d'adsorption des cations ;

On peut déterminer une énergie totale $\Delta E_{tot}$ pour désolvater ledit couple anion-cation d'un solvant et insérer ledit couple dans deux électrodes d'un supercondensateur, en réalisant les étapes suivantes :

- on détermine une énergie de désolvatation ($\Delta E_{desolv}$) dudit couple anion-cation ;

- on détermine une énergie de dissociation ($\Delta E_{diss}$) dudit couple anion-cation ;

- on détermine l'énergie d'adsorption ($\Delta E_{ads}$) dudit couple anion-cation au moyen du procédé selon l'invention ;

- on détermine le changement d'énergie totale ($\Delta E_{tot}$) en sommant l'énergie de désolvatation, l'énergie de dissociation et l'énergie d'adsorption.

Selon l'invention, on peut déterminer l'énergie de désolvatation ($\Delta E_{desolv}$) en réalisant une première simulation dynamique moléculaire pour calculer l'énergie totale moyenne de la phase condensée à une température donnée, et une seconde simulation dynamique moléculaire pour calculer l'énergie totale moyenne pour un seul couple d'ion ; et on peut déterminer l'énergie de dissociation ($\Delta E_{diss}$) en déterminant l'énergie du couple anion-cation, l'énergie du cation et l'énergie de l'anion.

Selon un mode de réalisation, on réalise un criblage de matériaux constituant les électrodes d'un supercondensateur, en réalisant les étapes suivantes :

- on choisit un couple anion-cation pour le liquide ionique ;

- on détermine l'énergie totale $\Delta E_{tot}$ pour ces ions pour différentes tailles de pore des électrodes au moyen du procédé selon l'invention ;

- on détermine la taille de pore permettant d'obtenir une capacité maximale en choisissant la taille de pore correspondant au minimum d'énergie totale $\Delta E_{tot}$.

On peut réaliser le criblage de couples cation-anion du liquide ionique d'un supercondensateur, en réalisant les étapes suivantes :

- on choisit une taille de pore pour les électrodes ;

- on détermine l'énergie totale $\Delta E_{tot}$ pour différents couple anion-cation pour ladite taille de pore, au moyen du procédé selon l'invention ;

- on sélectionne le couple anion-cation permettant d'obtenir une capacité maximale en choisissant le couple ayant l'énergie totale ($\Delta E_{tot}$) minimale pour ladite taille de pore.

D'autres caractéristiques et avantages du procédé selon l'invention, apparaîtront à la lecture de la description ci-après d'exemples non limitatifs de réalisations, en se référant aux figures annexées et décrites ci-après.

**Présentation succincte des figures**

- La figure 1 illustre la stratégie qui permet de calculer l'énergie totale nécessaire pour enlever un cation et un anion de leur phase condensée et de les adsorber dans les pores de l'électrode, en utilisant un cycle thermodynamique qui permet de couper $\Delta E_{tot}$ en trois termes d'énergie dont la somme est égale à $\Delta E_{tot}$.

- La figure 2 illustre les deux boites de simulation utilisées pour chaque électrode : dans chaque boite de simulation l'électrode est dupliquée en prenant sa charge opposée.

- La figure 3 illustre en détail une boîte de simulation pour calculer l'enthalpie d'adsorption, contenant deux nanotubes de charges opposées.

- La figure 4 montre des courbes d'énergie d'adsorption, de Van der Waals et électrostatique pour les cations TEA et EMIM et les anions BF4 et TSFI en fonction de la taille du pore de l'électrode.

- La figure 5 montre une courbe de capacité normalisée (courbe la plus à droite) et une courbe d'énergie totale (courbe la plus à gauche) pour le couple EMI/TFSI.

**Description détaillée du procédé**

L'invention est un procédé pour simuler les interactions électrostatiques (coulombiennes) entre un adsorbant électriquement chargé et un adsorbat électriquement chargé. Exemples :

- ion dans une zéolithe

- ion dans un MOF (« metal organic framework »)

- ion dans un nanotube

- protéine dans un enzyme

En particulier, l'invention est un procédé pour déterminer l'énergie totale pour désolvater un couple anion-cation d'un liquide ionique et pour insérer ce couple dans l'électrode d'un supercondensateur.

Comme l'illustre la figure 1, on utilise un cycle thermodynamique qui permet de décomposer l'énergie totale $\Delta E_{tot}$ en trois termes d'énergie dont la somme est égale à $\Delta E_{tot}$.

Le procédé comporte alors les étapes suivantes :

1. On détermine l'énergie de désolvatation ($\Delta E_{desolv}$)

2. On détermine l'énergie de dissociation ($\Delta E_{diss}$)

3. On détermine l'énergie d'adsorption ($\Delta E_{ads}$)

1. Détermination de l'énergie de désolvatation ($\Delta E_{desolv}$)

L'énergie de désolvatation correspond à l'énergie à apporter pour faire sortir un couple de liquide ionique (cation+anion) de la phase condensée (liquide ionique pur ou dissout dans un solvant tel que l'acétonitrile) à la phase gaz.

Pour la calculer, deux simulations sont requises :

i) on réalise une simulation dynamique moléculaire pour calculer l'énergie totale moyenne de la phase condensée (avec n pairs d'ions) à une température donnée : E1. Une telle technique est décrite par exemple dans les documents suivants :

- Allen, M. P.; Tildesley, D. J. Computer simulation of liquids; Oxford: Clarendon press, Ed.; 1987th ed.; Oxford University Press: Oxford, 1987.

- Frenkel, D.; Smit, B. Understanding Molecular Simulation; 2nd ed.; Academic Press: London, 2002; p. 638.

ii) on réalise une simulation de dynamique moléculaire pour calculer l'énergie totale moyenne pour un seul couple d'ion (cation+anion) : E2

L'énergie de désolvatation est alors la différence entre E1 divisé par le nombre de couples (n) et E2, c'est-à-dire : $AE_{desolv}$ = E1/n - E2.

Une telle technique est décrite par exemple dans les documents suivants :

- Shimizu, K.; Tariq, M.; Costa Gomes, M. F.; Rebelo, L. P. N.; Canongia Lopes, J. N. The journal of physical chemistry. B 2010, 114, 5831-4

2. Détermination de l'énergie de dissociation ($\Delta E_{diss}$)

L'énergie de dissociation représente l'énergie nécessaire pour dissocier le cation et l'anion. Elle est calculée à partir de trois calculs quantiques :

i) l'énergie du couple (pair) cation + anion : E3

ii) l'énergie du cation : E4

iii) l'énergie de l'anion : E5

Ensuite, on écrit : $\Delta E_{diss}$ = E3 - E4 - E5

Une telle technique est décrite par exemple dans les documents suivants :

- Fernandes, A. M.; Rocha, M. A. A.; Freire, M. G.; Marrucho, I. M.; Coutinho, J. A. P.; Santos, L. M. N. B. F. The journal of physical chemistry. B 2011, 115, 4033-41.

<u>3. Détermination de l'énergie d'adsorption ($\Delta E_{ads}$)</u>

L'énergie d'adsorption est l'énergie libérée quand on fait rentrer l'ion dans les pores de l'électrode (représenté par des nanotubes de carbone selon l'invention).

L'énergie d'adsorption est calculée au moyen d'une simulation moléculaire, où les interactions électrostatiques de courte et longue portée sont calculée avec la méthode d'Ewald, de la façon suivante :

$$\Delta E_{ads} = E_{électrode+ion} - E_{électrode} - E_{ion}$$

où $E_{électrode+ion}$, $E_{électrode}$ et $E_{ion}$ correspondent respectivement à l'énergie de l'électrode et de l'ion, à l'énergie de électrode seule et à l'énergie de l'ion seul. Cette énergie est l'énergie calculée avec un champ de force dont les paramètres ont été optimisés pour bien décrire les liquides ioniques. Ce champ de force peut s'appuyer sur les références suivantes:

- Canongia Lopes, J. N.; Deschamps, J.; Padua, A. A. H. The Journal of Physical Chemistry B 2004, 108, 11250.

- Canongia Lopes, J. N.; Padua, A. A. H. The Journal of Physical Chemistry B 2006, 110, 19586-19592.

- Canongia Lopes, J. N.; Padua, A. A. H. The Journal of Physical Chemistry B 2004, 108, 16893-16898.

- De Andrade, J.; Bo, E. S.; Stassen, H. Journal of Physical Chemistry B 2002, 3546-3548.

- Kaminski, G. A.; Jorgensen, W. L. Journal of the Chemical Society, Perkin Transactions 21999, 2365-2375

Chaque énergie potentielle ($E^{pot}$) peut être décomposée en deux termes principaux : l'énergie intramoléculaire ($E_{intra}$) et l'énergie intermoléculaire ($E_{inter}$). Par exemple :

$$E_{electrode+ion}^{pot} = E_{electrode+ion}^{Intra} + E_{electrode+ion}^{Inter} = E_{electrode+ion}^{Intra} + E_{electrode+ion}^{VanderWaals} + E_{electrode+ion}^{électrostatiques}$$

Le premier terme ($E^{intra}$) prend en compte les interactions entre atomes liés par une liaison, un angle ou un angle dièdre. Le deuxième terme ($E^{inter}$) contient les interactions dites non-liantes : Van der Waals et électrostatiques.

D'une façon générale, on définit l'énergie potentielle d'une espèce (ion, électrode, couple d'ions) par:

$$E^{pot} = E^{intra} + E^{inter}$$

$$E^{intra} = E^{liaisons} + E^{angles} + E^{dièdres} = \sum_{liaisons} \frac{k_i}{2}(l_i - l_{i,0})^2 + \sum_{angles} \frac{k_i}{2}(\theta_i - \theta_{i,0})^2 + \sum_{dièdres} \frac{V_n}{2}(1 + cos(n\omega - \gamma))$$

$$E^{inter} = E^{VanderWaals} + E^{électrostatiques} = \sum_{i=1}^{N} \sum_{j=i+1}^{N} 4\varepsilon_{ij}\left[\left(\frac{\sigma_{ij}}{r_{ij}}\right)^{12} - \left[\left(\frac{\sigma_{ij}}{r_{ij}}\right)^{6}\right]\right] + E^{électrostatiques}$$

Avec :

$l_i$ = longueur de la liaison $i$

$l_{i,0}$ = distance de référence pour cette liaison

$\theta_i$ = grandeur de l'angle $i$

$\theta_{i,0}$ = référence pour cette angle

$\omega$ = angle dièdre

$V_n$ = constante pour l'angle dièdre

$\gamma$ = constante pour l'angle dièdre

$\varepsilon_{ij}$ = énergie de référence pour un couple d'atomes $i$ et $j$

$\sigma_{ij}$ = distance de référence pour un couple d'atomes $i$ et $j$

Cette définition est notamment décrite dans : Leach, A. R. Molecular Modelling: Principles and Applications; 2nd ed.; Prentice Hall, 2001.

Les interactions électrostatiques ont une (très) longue portée. En conséquence, même les atomes (ou corps) chargés et séparés par de longues distances subissent une interaction électrostatique. Pour prendre en compte cette interaction, la méthode Ewald est très couramment appliquée. La méthode Ewald est une méthode répandue en simulation moléculaire. Elle permet d'évaluer les interactions électrostatiques à courte distance dans un espace réel et à longue distance dans un espace réciproque, afin que la sommation converge vers une certaine valeur.

La méthode d'Ewald est une méthode de calcul des énergies d'interaction de systèmes périodiques et tout particulièrement les énergies électrostatiques. Les énergies électrostatiques comprenant à la fois des termes d'interactions de courtes et de longues portées, il est très intéressant de décomposer le potentiel d'interaction en termes de courte portée - dont la sommation se fait dans l'espace réel - et de longue portée - dont la sommation se fait dans l'espace de Fourier (espace réciproque). L'avantage de cette approche est la convergence rapide de la sommation dans l'espace de Fourier comparée à son équivalent dans l'espace réel lorsque les interactions se font à longue distance.

Tous les atomes du cation et anion ont une charge nette partielle. Chaque atome est donc considéré comme un "point charge". Ensuite chaque "point charge" dans le système contribue à l'énergie électrostatique totale, qui est calculée selon la formule suivante :

$$E_{elec} = \frac{1}{2} \sum_{i=1}^{N} \sum_{j=1}^{N} \left\{ \begin{array}{l} \sum_{|\mathbf{n}|=0}^{\infty} {}' \dfrac{q_i q_j}{4\pi\varepsilon_0} \dfrac{erfc(\alpha|\mathbf{r}_{ij}+\mathbf{n}|)}{|\mathbf{r}_{ij}+\mathbf{n}|} + \\[2ex] \sum_{k\neq 0} \dfrac{1}{\pi L^3} \dfrac{q_i q_j}{4\pi\varepsilon_0} \dfrac{4\pi^2}{k^2} \exp\left(-\dfrac{k^2}{4\alpha^2}\right) \cos(\mathbf{k}\cdot\mathbf{r}_{ij}) \\[2ex] -\dfrac{\alpha}{\sqrt{\pi}} \sum_{k=1}^{N} \dfrac{q_k^2}{4\pi\varepsilon_0} \end{array} \right\}$$

où

- le $1^{er}$ terme présente la contribution dans l'espace réel

- le $2^e$ terme présente la contribution dans l'espace réciproque

- le $3^e$ terme présente une correction pour les interactions entre chaque gaussien avec lui-même

Avec (définition des variables principales) :

$E_{elec}$: énergie électrostatique (interaction entre deux particules électriquement chargées)

$q_i$ ou $q_j$ : charge atomique nette des atomes $i$ et $j$.

$\mathbf{r}_{ij}$ : distance entre les atomes $i$ et $j$ portant une charge $q_i$ et $q$ respectivement

$N$ : nombre total d'atomes portant une charge (atomes de l'électrode + atomes du liquide ionique)

$\mathbf{n} = (n_x L, n_y L, n_z L)$ $n_x$, $n_y$, $n_z$ sont des entiers

$L$ : longueur de la boîte,

erfc : fonction d'erreur complémentaire: $erfc(x) = \dfrac{2}{\sqrt{\pi}} \int_x^\infty \exp(-t^2)dt$

$\alpha$ : définit la "largeur" de la fonction gaussienne : $f(x) = \dfrac{1}{\alpha\sqrt{2\pi}} \exp\left(-\dfrac{(x-\mu)^2}{2\alpha^2}\right)$

$\mathbf{k}$ : $2\pi\mathbf{n}/L$ (vecteur réciproque):

$k$ : la norme du vecteur $\mathbf{k}$

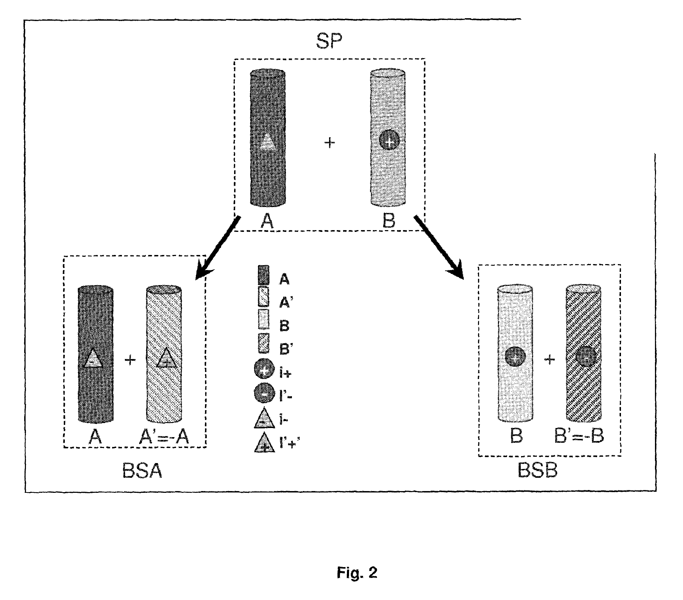

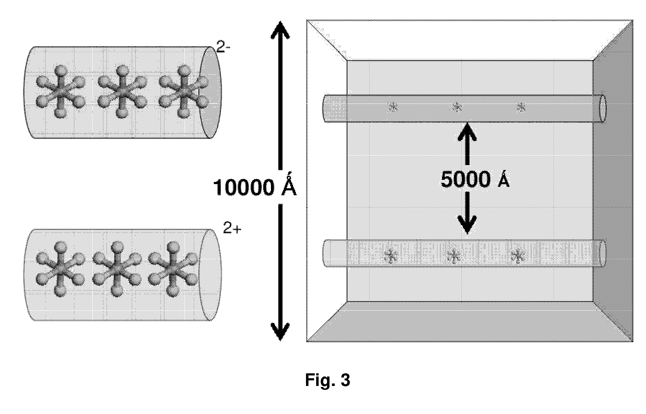

La méthode Ewald calcule la partie électrostatique. Toutefois, cette méthode nécessite que la charge totale dans la boîte de simulation soit zéro (neutre), sinon le deuxième terme dans l'équation (3) ne converge pas. La boite de simulation est une représentation, une maquette, de l'environnement physique dans lequel on étudie les phénomènes.
Or, une simulation directe du système physique, quand on insère un ion dans l'électrode, implique que : soit l'électrode seule est chargée, soit le système électrode+ion est chargé. Du coup, la méthode Ewald n'est plus applicable.
Pour s'affranchir de ce problème, le procédé selon l'invention se base sur une boite de simulation dans laquelle chaque électrode est dupliquée en prenant une charge exactement opposée, comme l'illustre la figure 2.
Ainsi, selon l'invention, on construit, à partir du système physique (SP) deux boites de simulations BSA et BSB.
L'électrode positive A et l'anion (i-) dans le système physique, sont étudiés au sein d'une boite de simulation BSA comportant : l'électrode A et l'anion (i-), ainsi qu'une électrode A' de charge opposée et un ion (i'+) de charge opposée.
L'électrode négative B et le cation (i+) dans le système physique, sont étudiés au sein d'une boite de simulation BSB comportant : l'électrode B et le cation (i+), ainsi qu'une électrode B' de charge opposée et un ion (i'-) de charge opposée.
Afin d'assurer une électroneutralité dans chaque boîte de simulation, le nombre de cations et cations avec charge opposée (cation') est identique, afin que la charge totale soit zéro. De la même façon les charges de l'électrode A et A' s'annulent.
Ainsi, la méthode Ewald peut être appliquée.
Pour obtenir, l'énergie d'adsorption de l'anion dans l'électrode A, on divise par 2 l'énergie d'adsorption ($\Delta E_{ads}$, = $E_{électrode+ion}$ - $E_{électrode}$ - $E_{ion}$) calculée dans la boite de simulation BSA.
Pour obtenir, l'énergie d'adsorption du cation dans l'électrode B, on divise par 2 l'énergie d'interaction calculée dans la boite de simulation BSB.
De façon, plus détaillée, on modélise chaque électrode par des nanotubes de longueur infinie, séparés d'une distance $d$, et ayant exactement la même charge électrique absolue, mais opposée afin que la charge totale soit zéro. La distance d est choisie de façon à ce que les deux nanotubes soient suffisamment séparés pour que les interactions électrostatiques et Van der Waals deviennent négligeables. Puis, chaque nanotube est rempli avec exactement le même nombre (1, 2, 3...) d'ions de la même nature, mais avec des charges atomiques opposées. Ainsi, on peut mettre un ou plusieurs anions dans le nanotube avec une charge positive et dans le second nanotube une charge négative avec le même anion, mais avec ses charges atomiques opposées pour devenir un cation.
Cette représentation est illustrée sur la figure 3 : le nanotube du haut porte une charge de 2-, le nanotube du bas porte la même charge absolue, mais opposée (2+). Le nanotube du bas est chargé avec trois anions [PF6]- (hexafluorophosphate) en conséquence, la charge totale de ce sous-système vaut -1, tandis que le nanotube du haut est chargé avec trois anions avec charges opposées [PF6]+ rendant la charge totale pour ce sous-système égale à +1 ; la charge totale de la boîte de simulation vaut zéro.
Notons que nous avons uniquement opposé les charges atomiques : tous les autres paramètres du champ de force (ensemble d'équations et de paramètres décrivant l'énergie potentielle d'un système de particules) restent identiques, afin que l'énergie totale interne soit exactement la même pour les deux espèces (anions et cations). On a donc toujours une électro neutralité pour le système total, car les nanotubes ont une charge opposée, comme les deux ions. La méthode Ewald peut donc être utilisée pour évaluer les interactions électrostatiques.

**L'utilisation de cette approche apporte plusieurs avantages :**

1. On a toujours une électroneutralité pour le système total, car les nanotubes ont une charge opposée, comme les deux ions. La méthode Ewald peut donc être utilisée pour évaluer les interactions électrostatiques.

2. La charge totale d'un nanotube rempli avec un certain nombre d'ions n'a pas besoin d'être nulle, car cette charge nette sera compensée avec la charge de l'autre nanotube avec le même nombre d'ions (mais avec charge opposée). Cette approche permet de :

a) varier le nombre d'ions dans chaque nanotube indépendamment de la charge du nanotube. Ainsi, à charge électrique constante du nanotube, on peut étudier le nombre maximal d'ion (anions ou cations) qui peut rentrer dans le nanotube;

b) varier la charge du nanotube indépendamment du nombre d'ions inséré. Si le diamètre du nanotube est « trop » petit par rapport à la taille de l'ion, pour le faire rentrer, en augmentant la charge du nanotube les interactions électrostatiques peuvent compenser l'énergie de déformation de l'ion

3. Comme la simulation du système conduit à un calcul correspondant au double de l'énergie d'interaction moyenne entre ion et nanotube (une énergie moyenne pour chaque nanotube), on améliore également la statistique pour calculer l'énergie d'adsorption moyenne.

**Exemples**

Énergies d'adsorption

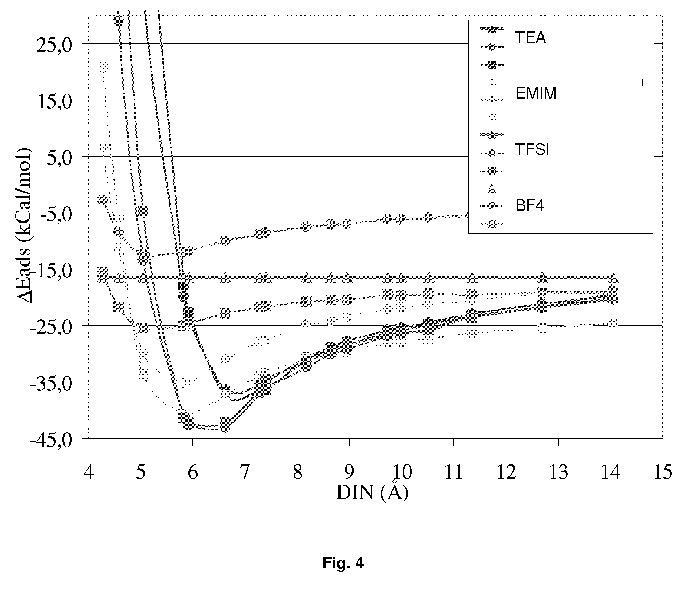

Dans la figure 4, l'énergie d'adsorption ($AE_{ads}$) a été décomposée avec une contribution venant des interactions de Van der Waals ($E_{vdw}$) et des interactions électrostatiques ($E_{electro}$). Les interactions de Van der Waals ($E_{vdw}$) sont représentées par des ronds les interactions électrostatiques ($E_{electro}$) sont représentées par des triangles, et l'énergie d'adsorption ($AE_{ads}$) est représentée par des carrés.

Ces énergies sont tracées en fonction du diamètre interne du nanotube (DIN), ce diamètre modélisant la taille du pore de l'électrode, pour les cations tetraéthylammonium ($TEA^+$) et éthylméthylimidazolium ($EMIM^+$), respectivement pour les anions tétrafluoroborate ($BF4^-$) et bis(trifluorométhanesulfonyl)imide ($TFSI^-$).

Les résultats montrent, pour une charge constante ($\pm 2e$ par nanotube), que la contribution électrostatique est indépendante de la taille du pore de l'électrode et indépendante de la nature de l'ion : elle est en fait entièrement déterminée par la charge (toujours $\pm 1e$) totale de l'ion et du nanotube (toujours $\pm 2e$).

En revanche, l'énergie d'adsorption montre une courbe avec un minimum et sa forme est dictée par la forme de la courbe qui représente la contribution de l'énergie Van der Waals.

Les minima des courbes de l'énergie d'adsorption présentés dans la figure 4 sont une fonction de la taille des ions. Les ions plus petits en taille géométrique ($BF_4$ et EMIM) arrivent encore à rentrer dans les nanotubes avec des petits diamètres (5 à 6 Å), c'est à dire que l'énergie d'adsorption est négative tant que le DIN est supérieur à environ 4.5 A°, tandis que les ions plus grands sont exclus, c'est à dire que l'énergie d'adsorption devient positive dès que le DIN est inférieur à environ 5.5 A° (TFSI) ou 6 A° (TEA) (figure 4).

On remarque également que l'énergie d'interaction (profondeur du puits) entre l'ion et l'hôte est plus grande pour les ions constitués d'un nombre d'atomes plus important (TFSI : 15 atomes ; EMIM 19 atomes ; $NEt_4$ 29 atomes) que pour les ions à nombre d'atomes faible ($BF_4$ : 5 atomes). Cette interaction est plus élevée, car chaque atome contribue à la somme totale des interactions Van der Waals. Notons que la forme de l'ion joue également un rôle important : un ion avec une géométrie « allongée » comme TFSI a une interaction plus forte avec un cylindre (nanotube) qu'un ion avec une géométrie plus sphérique comme le $NEt_4$.

**Exemples d'application du procédé**

En utilisant le procédé selon l'invention, pour déterminer l'énergie totale ($\Delta E_{tot}$) de désolvatation d'un couple d'anion+cation du liquide ionique et d'adsorption dans les pores de l'électrode, prenant en compte les énergies électrostatiques, il est possible de prédire la capacité du supercondensateur en fonction de la taille des pores des électrodes ou en fonction des couples anion-cation dans le liquide ionique.

On peut alors déterminer une taille de pore des électrodes permettant d'obtenir une capacité maximale dudit supercondensateur pour un liquide ionique donné.

On peut également déterminer des couples anion-cation permettant d'obtenir une capacité maximale dudit supercondensateur pour une taille donnée des pores des électrodes.

Prédiction de la capacité du supercondensateur en fonction de la taille des pores

On constate expérimentalement qu'une courbe (mesurée) de capacité et une courbe représentant l'énergie totale ($\Delta E_{tot}$) déterminée selon l'invention (prenant en compte la distribution des tailles de pores), ont leur maximum, respectivement minimum, sensiblement pour une même taille de pore.

Ainsi, la courbe représentant l'énergie totale est un très bon indicateur pour estimer la taille de pore optimale de l'électrode.

Le procédé pour réaliser un criblage de matériaux constituant les électrodes d'un supercondensateur, comporte alors les étapes suivantes :

- on choisit un couple anion-cation pour le liquide ionique (électrolyte du supercondensateur) ;

- on détermine l'énergie totale ($\Delta E_{tot}$) pour ces ions pour différentes tailles de pore des électrodes au moyen du procédé selon l'invention ;

- on détermine la taille de pore permettant d'obtenir une capacité maximale en choisissant la taille de pore correspondant au minimum d'énergie ($\Delta E_{tot}$).

*Exemple*

On choisit le couple anion-cation suivant : EMIM/TFSI

On détermine l'énergie totale de ces ions pour différentes tailles de pore des électrodes, en appliquant le procédé selon l'invention.

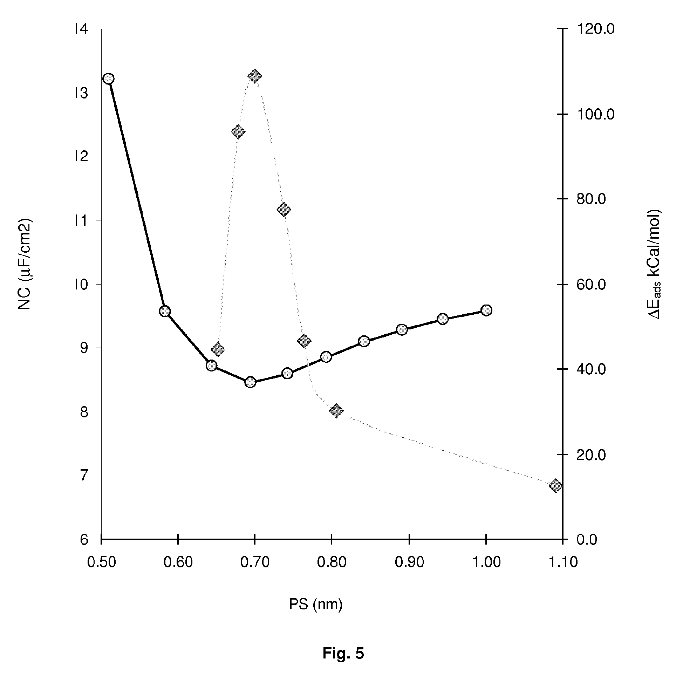

Les courbes de l'énergie totale montrent une analogie avec la capacité normalisée, mesurée expérimentalement par P. Simon et al. (Science (New York, N.Y.) 2006, 313, 1760-3). Ceci est visible sur la figure 5 qui illustre d'une part l'évolution de la capacité normalisée (NC - courbe avec les losanges) du super condensateur (avec le couple EMIM/TFSI) mesurée expérimentalement en fonction de la taille des pores (PS), et d'autre part, l'évolution de l'énergie totale ($\Delta E_{tot}$ - courbe avec des ronds) en fonction de la taille des pores.

On observe que le maximum de la courbe "capacité" et le minimum de la courbe $\Delta E_{tot}$ sont atteints pour une taille de pore sensiblement égale à 0,7 nm.

Cette taille de pore est donc la taille de pore optimale pour un supercondensateur fonctionnant avec le couple EMIM/TFSI.

Prédiction de la capacité du supercondensateur en fonction des couples anion-cation

Inversement, pour déterminer des couples anion-cation permettant d'obtenir une capacité maximale dudit supercondensateur pour une taille donnée des pores des électrodes, le procédé comporte les étapes suivantes :

- on choisit une taille de pore pour les électrodes ;

- on détermine l'énergie totale ($\Delta E_{tot}$) pour différents couple anion-cation pour ladite taille de pore, au moyen du procédé selon l'invention ;

- on sélectionne le couple anion-cation permettant d'obtenir une capacité maximale en choisissant le couple ayant l'énergie totale ($\Delta E_{tot}$) minimale pour ladite taille de pore.

**Revendications**

1. Procédé pour déterminer l'énergie d'adsorption entre un adsorbant électriquement chargé et un adsorbat électriquement chargé, prenant en compte des interactions électrostatiques entre l'adsorbat et l'adsorbant, en réalisant les étapes suivantes :

   - on construit une boite de simulation (BSA) comportant ledit adsorbant (A, B) et ledit adsorbat (i+, i-), ainsi qu'un autre adsorbant (A', B') du même type mais de charge opposée et un autre adsorbat (i'+, i'-) du même type mais de charge opposée, de façon à ce que ladite boite de simulation ait une charge nulle ;
   - on détermine l'énergie d'adsorption desdits adsorbats (i+, i- et i') dans ladite première boite de simulation (BSA) au moyen d'une simulation moléculaire et de la méthode Ewald, et on en déduit l'énergie d'adsorption dudit adsorbat (i+, i-) sur ledit adsorbant (A, B).

2. Procédé selon la revendication 1, dans lequel l'adsorbant est une zéolithe, ou un nanotube ou une enzyme ou une électrode (A, B), et l'adsorbat est un ion (i+,i-) ou une protéine.

3. Procédé selon la revendication 2, dans lequel on détermine l'énergie d'adsorption d'un couple anion-cation d'un liquide ionique, sur deux électrodes d'un supercondensateur, en prenant en compte des interactions électrostatiques entre les ions et l'électrode en réalisant les étapes suivantes :

   - on construit une première boite de simulation (BSA) comportant ladite électrode positive A et au moins un anion, ainsi qu'une électrode A' de charge opposée et au moins un ion de charge opposée, de façon à ce que ladite boite de simulation ait une charge nulle ;
   - on construit une seconde boite de simulation (BSB) comportant ladite électrode négative B et au moins un cation, ainsi qu'une électrode B' de charge opposée et au moins un ion de charge opposée, de façon à ce que ladite boite de simulation ait une charge nulle ;
   - on détermine une contribution électrostatique de l'énergie d'adsorption desdits ions dans ladite première boite de simulation (BSA) au moyen de la méthode Ewald, et on en déduit l'énergie d'adsorption des anions ;
   - on détermine une contribution électrostatique de l'énergie d'adsorption desdits ions dans ladite seconde boite de simulation (BSB) au moyen de la méthode Ewald, et on en déduit l'énergie d'adsorption des cations.

4. Procédé selon la revendication 3, dans lequel on détermine une énergie totale $\Delta E_{tot}$ pour désolvater ledit couple anion-cation d'un solvant et insérer ledit couple dans deux électrodes d'un supercondensateur, en réalisant les étapes suivantes :

   - on détermine une énergie de désolvatation ($\Delta E_{desolv}$) dudit couple anion-cation ;
   - on détermine une énergie de dissociation ($\Delta E_{diss}$) dudit couple anion-cation ;
   - on détermine l'énergie d'adsorption ($\Delta E_{ads}$) dudit couple anion-cation au moyen du procédé selon la revendication 3;
   - on détermine le changement d'énergie totale ($\Delta E_{tot}$) en sommant l'énergie de désolvatation, l'énergie de dissociation et l'énergie d'adsorption.

5. Procédé selon la revendication 4, dans lequel :

   - on détermine l'énergie de désolvatation ($\Delta E_{desolv}$) en réalisant une première simulation dynamique moléculaire pour calculer l'énergie totale moyenne de la phase condensée à une température donnée, et une seconde simulation dynamique moléculaire pour calculer l'énergie totale moyenne pour un seul couple d'ion ; et
   - on détermine l'énergie de dissociation ($\Delta E_{diss}$) en déterminant l'énergie du couple anion-cation, l'énergie du cation et l'énergie de l'anion.

6. Procédé selon l'une des revendications 3 à 5, dans lequel on réalise un criblage de matériaux constituant les électrodes d'un supercondensateur, en réalisant les étapes suivantes :

   - on choisit un couple anion-cation pour le liquide ionique ;
   - on détermine l'énergie totale $\Delta E_{tot}$ pour ces ions pour différentes tailles de pore des électrodes au moyen du procédé selon l'une des revendications 4 et 5 ;
   - on détermine la taille de pore permettant d'obtenir une capacité maximale en choisissant la taille de pore correspondant au minimum d'énergie totale $\Delta E_{tot}$.

7. Procédé selon l'une des revendications 3 à 5, dans lequel on réalise un criblage de couples cation-anion du liquide ionique d'un supercondensateur, en réalisant les étapes suivantes :

   - on choisit une taille de pore pour les électrodes ;
   - on détermine l'énergie totale $\Delta E_{tot}$ pour différents couple anion-cation pour ladite taille de pore, au moyen du procédé selon l'une des revendications 4 et 5 ;
   - on sélectionne le couple anion-cation permettant d'obtenir une capacité maximale en choisissant le couple ayant l'énergie totale ($\Delta E_{tot}$ ) minimale pour ladite taille de pore.

**Patentansprüche**

1. Verfahren zur Bestimmung der Adsorptionsenergie zwischen einem elektrisch geladenen Adsorbens und einem elektrisch geladenen Adsorbat unter Berücksichtigung der elektrostatischen Wechselwirkungen zwischen dem Adsorbat und dem Adsorbens, wobei die folgenden Schritte durchgeführt werden:

   - Konstruieren einer Simulationsbox (BSA), umfassend das Adsorbens (A, B) und das Adsorbat (i+, i-) sowie ein anderes Adsorbens (A', B') gleichen Typs aber entgegen gesetzter Ladung und ein anderes Adsorbat (i'+, i'-) gleichen Typs aber entgegen gesetzter Ladung, damit die Simulationsbox eine Ladung null aufweist;
   - Bestimmen der Adsorptionsenergie der Adsorbate (i+, i- und i') in der ersten Simulationsbox (BSA) mithilfe einer molekularen Simulation und der Ewald-Methode, und davon Ableiten der Adsorptionsenergie des Adsorbats (i+, i-) auf dem Adsorbens (A, B).

2. Verfahren nach Anspruch 1, wobei das Adsorbens ein Zeolith oder ein Nanoröhrchen oder ein Enzym oder eine Elektrode (A, B) ist, und das Adsorbat ein Ion (i+, i-) oder ein Protein ist.

3. Verfahren nach Anspruch 2, wobei die Adsorptionsenergie eines Anionen-Kationen-Paars einer ionischen Flüssigkeit an zwei Elektroden eines Superkondensators unter Berücksichtigung der elektrostatischen Wechselwirkungen zwischen den Ionen und der Elektrode bestimmt wird, indem die folgenden Schritte durchgeführt werden:

   - Konstruieren einer ersten Simulationsbox (BSA), umfassend die positive Elektrode A und mindestens ein Anion sowie eine Elektrode A' entgegen gesetzter Ladung und mindestens ein Ion entgegen gesetzter Ladung, damit die Simulationsbox eine Ladung null aufweist;
   - Konstruieren einer zweiten Simulationsbox (BSB), umfassend die negative Elektrode B und mindestens ein Kation sowie eine Elektrode B' entgegen gesetzter Ladung und mindestens ein Ion entgegen gesetzter Ladung, damit die Simulationsbox eine Ladung null aufweist;
   - Bestimmen eines elektrostatischen Beitrags der Adsorptionsenergie der Ionen in der ersten Simulationsbox (BSA) mithilfe der Ewald-Methode, und davon Ableiten der Adsorptionsenergie der Anionen;
   - Bestimmen eines elektrostatischen Beitrags der Adsorptionsenergie der Ionen in der zweiten Simulationsbox (BSB) mithilfe der Ewald-Methode, und davon Ableiten der Adsorptionsenergie der Kationen;

4. Verfahren nach Anspruch 3, wobei eine Gesamtenergie $\Delta E_{tot}$ zum Desolvatisieren des Anionen-Kationen-Paars eines Lösungsmittels bestimmt wird und Einsetzen des Paars in zwei Elektroden eines Superkondensators, indem die folgenden Schritte durchgeführt werden:

   - Bestimmen einer Desolvatisierungsenergie ($\Delta E_{desolv}$) des Anionen-Kationen-Paars;
   - Bestimmen einer Dissoziationsenergie ($\Delta E_{diss}$) des Anionen-Kationen-Paars;
   - Bestimmen der Adsorptionsenergie ($\Delta E_{ads}$) des Anionen-Kationen-Paars mithilfe des Verfahrens nach Anspruch 3;
   - Bestimmen der Veränderung der Gesamtenergie ($\Delta E_{tot}$) indem die Desolvatisierungsenergie, die Dissoziationsenergie und die Adsorptionsenergie addiert werden.

5. Verfahren nach Anspruch 4, wobei:

   - die Desolvatisierungsenergie ($\Delta E_{desolv}$) bestimmt wird, indem eine erste dynamische molekulare Simulation zur Berechnung der mittleren Gesamtenergie der kondensierten Phase bei einer gegebenen Temperatur und eine zweite dynamische molekulare Simulation zur Berechnung der mittleren Gesamtenergie für ein einziges Ionenpaar durchgeführt werden; und
   - die Dissoziationsenergie ($\Delta E_{diss}$) bestimmt wird, indem die Energie des Anionen-Kationen-Paars, die Energie des Kations und die Energie des Anions bestimmt werden.

6. Verfahren nach einem der Ansprüche 3 bis 5, wobei ein Screening von Materialien durchgeführt wird, die die Elektroden eines Superkondensators bilden, indem die folgenden Schritte durchgeführt werden:

   - Auswählen eines Anionen-Kationen-Paars für die ionische Flüssigkeit;
   - Bestimmen der Gesamtenergie $\Delta E_{tot}$ für diese Ionen für verschiedene Porengrößen der Elektroden mithilfe des Verfahrens nach einem der Ansprüche 4 und 5;
   - Bestimmen der Porengröße, die es ermöglicht, eine maximale Kapazität zu erhalten, indem die Porengröße ausgewählt wird, die der minimalen Gesamtenergie $\Delta E_{tot}$ entspricht.

7. Verfahren nach einem der Ansprüche 3 bis 5, wobei ein Screening von Kationen-Anionen-Paaren der ionischen Flüssigkeit eines Superkondensators durchgeführt wird, indem die folgenden Schritte durchgeführt werden:

- Auswählen einer Porengröße für die Elektroden;
- Bestimmen der Gesamtenergie $\Delta E_{tot}$ für verschiedene Anionen-Kationen-Paare für die Porengröße mithilfe des Verfahrens nach einem der Ansprüche 4 und 5;
- Auswählen des Anionen-Kationen-Paars, das es ermöglicht, eine maximale Kapazität zu erhalten, indem das Paar gewählt wird, das die minimale Gesamtenergie ($\Delta E_{tot}$) für die Porengröße aufweist.

**Claims**

1. A method for determining the adsorption energy between an electrically charged adsorbent and an electrically charged adsorbate, taking account of electrostatic interactions between the adsorbate and the adsorbent, by carrying out the following stages:

- constructing a simulation box (BSA) containing said adsorbent (A, B) and said adsorbate (i+, i-), and another adsorbent (A', B') of same type but of opposite charge and another adsorbate (i'+, i'-) of same type but of opposite charge, so that said simulation box has zero charge;
- determining the adsorption energy of said adsorbates (i+, i- and i') in said first simulation box (BSA) using a molecular simulation and the Ewald method, and deducing therefrom the adsorption energy of said adsorbate (i+, i-) on said adsorbent (A, B).

2. A method as claimed in claim 1, wherein the adsorbent is a zeolite, or a nanotube or an enzyme or an electrode (A, B), and the adsorbate is an ion (i+, i-) or a protein.

3. A method as claimed in claim 2, wherein the adsorption energy of an anion-cation pair of an ionic liquid is determined on two electrodes of a supercapacitor by taking into account electrostatic interactions between the ions and the electrode, by carrying out the following stages:

- constructing a first simulation box (BSA) comprising said positive electrode A and at least one anion, as well as an electrode A' of opposite charge and at least one ion of opposite charge, so that said simulation box has zero charge;
- constructing a second simulation box (BSB) comprising said negative electrode B and at least one cation, as well as an electrode B' of opposite charge and at least one ion of opposite charge, so that said simulation box has zero charge;
- determining an electrostatic contribution of the adsorption energy of said ions in said first simulation box (BSA) using the Ewald method, and deducing therefrom the adsorption energy of the anions;
- determining an electrostatic contribution of the adsorption energy of said ions in said second simulation box (BSB) using the Ewald method, and deducing therefrom the adsorption energy of the cations.

4. A method as claimed in claim 3, wherein a total energy $\Delta E_{tot}$ for desolvating said anion-cation pair of a solvent and for inserting said pair into two electrodes of a supercapacitor is determined by carrying out the following stages:

- determining a desolvation energy ($\Delta E_{desolv}$) of said anion-cation pair;
- determining a dissociation energy ($\Delta E_{diss}$) of said anion-cation pair;
- determining the adsorption energy ($\Delta E_{ads}$) of said anion-cation pair by means of the method as claimed in claim 3;
- determining the change in total energy ($\Delta E_{tot}$) by summing the desolvation energy, the dissociation energy and the adsorption energy.

5. A method as claimed in claim 4, wherein:

- desolvation energy ($\Delta E_{desolv}$ is determined by performing a first molecular dynamic simulation to calculate the average total energy of the condensed phase at a given temperature, and a second molecular dynamic simulation to calculate the average total energy for a single ion pair, and

- dissociation energy ($\Delta E_{diss}$) is determined by determining the energy of the anion-cation pair, the energy of the cation and the energy of the anion.

6. A method as claimed in any one of claims 3 to 5, wherein screening of the materials making up the electrodes of a supercapacitor is performed by carrying out the following stages:

- selecting an anion-cation pair for the ionic liquid;
- determining the total energy $\Delta E_{tot}$ for these ions for different pore sizes of the electrodes by means of the method as claimed in any one of claims 4 and 5;
- determining the pore size allowing to obtain a maximum capacitance by selecting the pore size corresponding to the minimum total energy $\Delta E_{tot}$.

7. A method as claimed in any one of claims 3 to 5, wherein screening of cation-anion pairs of the ionic liquid of a supercapacitor is performed by carrying out the following stages:

- selecting a pore size for the electrodes;
- determining the total energy $\Delta E_{tot}$ for different anion-cation pairs for said pore size by means of the method as claimed in any one of claims 4 and 5;
- selecting the anion-cation pair allowing to obtain a maximum capacitance by selecting the pair having the minimum total energy ($\Delta E_{tot}$) for said pore size.

$$\Delta E_{tot} = \Delta E_{desolv} + \Delta E_{diss} + \Delta E_{ads\text{-}an} + \Delta E_{ads\text{-}cat}$$

$\Delta E_{desolv}$

$\Delta E_{diss}$

$\Delta E_{ads\text{-}an}$

$\Delta E_{ads\text{-}cat}$

+

Fig. 1

EP 2 912 674 B1

Fig. 2

16

Fig. 3

**Fig. 4**

**Fig. 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **T. KÖDDERMANN ; D. PASCHEK ; R. LUDWIG.** *ChemPhysChem,* 2008, vol. 9, 549
- **SHIMIZU, K. ; TARIQ, M. ; COSTA GOMES, M. F. ; REBELO, L. P. N. ; CANONGIA LOPES, J. N.** *The journal of Physical Chemistry. B,* 2010, vol. 114, 5831-4
- **SHIM Y et al.** *ACS Nano,* 27 Avril 2010, vol. 4 (4), ISSN 1936-0851, 2345-2355
- **ALLEN, M. P. ; TILDESLEY, D. J.** Computer simulation of liquids. Clarendon press, 1987
- **FRENKEL, D. ; SMIT, B.** Understanding Molecular Simulation. Academic Press, 2002, 638
- **SHIMIZU, K. ; TARIQ, M. ; COSTA GOMES, M. F. ; REBELO, L. P. N. ; CANONGIA LOPES, J. N.** *The journal of physical chemistry. B,* 2010, vol. 114, 5831-4
- **FERNANDES, A. M. ; ROCHA, M. A. A. ; FREIRE, M. G. ; MARRUCHO, I. M. ; COUTINHO, J. A. P. ; SANTOS, L. M. N. B. F.** *The journal of physical chemistry. B,* 2011, vol. 115, 4033-41
- **CANONGIA LOPES, J. N. ; DESCHAMPS, J. ; PADUA, A. A. H.** *The Journal of Physical Chemistry B,* 2004, vol. 108, 11250
- **CANONGIA LOPES, J. N. ; PADUA, A. A. H.** *The Journal of Physical Chemistry B,* 2006, vol. 110, 19586-19592
- **CANONGIA LOPES, J. N. ; PADUA, A. A. H.** *The Journal of Physical Chemistry B,* 2004, vol. 108, 16893-16898
- **DE ANDRADE, J. ; BO, E. S. ; STASSEN, H.** *Journal of Physical Chemistry B,* 2002, 3546-3548
- **KAMINSKI, G. A. ; JORGENSEN, W. L.** *Journal of the Chemical Society, Perkin Transactions,* vol. 21999, 2365-2375
- **LEACH, A. R.** Molecular Modelling: Principles and Applications. Prentice Hall, 2001
- **P. SIMON et al.** *Science (New York, N.Y.,* 2006, vol. 313, 1760-3